# EUROPEAN PATENT SPECIFICATION

(11) **EP 3 099 353 B1**
(45) Date of publication and mention of the grant of the patent: **28.11.2018**
(21) Application number: 15743092.7
(22) Date of filing: 22.01.2015
(51) Int. Cl.: A61M 5/315, A61M 5/50, A61M 5/20, A61M 5/31, A61M 5/32

(54) **MEDICAMENT DISPENSING DEVICE**
MEDIKAMENTENAUSGABEVORRICHTUNG
DISPOSITIF DISTRIBUTEUR DE MÉDICAMENT

(30) Priority: 30.01.2014 US 201414168644
(43) Date of publication of application: 07.12.2016
(73) Proprietor: Medical Injection Devices Inc., Toronto, Ontario M2P 1V4 (CA)
(72) Inventor: SEGAL, Eric, Toronto, Ontario M2N 6S5 (CA)
(74) Representative: Patentanwälte Olbricht Buchhold Keulertz
(86) International application number: PCT/CA2015/050041
(87) International publication number: WO 2015/113149

(56) References cited:
- WO-A1-2010/127449
- WO-A1-2010/127449
- WO-A1-2011/039237
- WO-A1-2011/040861
- DE-A1- 3 609 555
- FR-A1- 2 639 832
- GB-A- 228 764
- US-A1- 2013 204 229
- US-A1- 2013 281 936
- US-B2- 7 678 084

## Description

### FIELD OF THE INVENTION

The present invention relates to a medicament dispenser and more particularly, the present invention relates to a structure for dispensing a medicament where the structure has a mechanical coupling member to substantially reduce jamming of the components normally attributable to such devices.

### BACKGROUND OF THE INVENTION

Single use single dose medicament delivery devices are widely known and are generally used to dispense medicaments such as epinephrine in an urgent manner to a user, the so called "epi pen" is a typical example of such arrangements. One of the significant disadvantages inherent with these devices relates to jamming. The existing devices often employ spring configurations for movement of key parts such as the needle as well as the barrel which assists in delivery of the medicament to the needle. In the scenario where a jam has occurred, the result can be fatal if the user cannot repair the device to function properly. In some instances, repair is impossible given urgent time constraints and the user is forced to dismantle or destroy the structure in order to gain access to the medication.

The above is possible where the user is not a child or a user otherwise not capable of achieving access. To address the limitations in the art, a variety of solutions have been advanced in the art. An example is provided in United States Patent No. 6,808,507, issued October 26, 2004, to Roser. In the arrangement discussed in this patent, a telescopic member is provided and a spring surrounds the needle. The arrangement is useful, however, there is still a possibility that the spring could be defective or otherwise fail, thus complicating delivery.

Botich et al., in United States Patent No. 6,039,713, issued March 21, 2000, teach a pre-filled retractable needle device. The device, as is common with most arrangements, includes reciprocating body members, springs, etc. In this instance the device has a number of movable parts which elevates the possibility for jamming or failure.

United States Patent No. 6,846,301, issued to Smith ct al., June 25, 2005, teach a disposable safety syringe with a vacuum system to withdraw the needle into the body after use. There is no provision for a mechanical linkage for quick delivery of the medicament.

PCT Application No. PCT/CA2010/000694, published as WO2010/127449, teaches a portable medicament dispensing device that employs a coupling for coupling the relative motion between reciprocating body members housing ancillary components and a barrel plunger associated with the syringe. The coupling include pivoting couplings and a rack and pinion system.

Given the extent of development in the prior art, there exists a need for an improved medicament dispenser which is efficient and reliable while maintaining a lower profile than those devices currently available.

The present invention provides a significantly improved arrangement which is devoid of the structural limitations inherent with the prior art.

### SUMMARY OF THE INVENTION

One object of one embodiment of the present invention is to provide an improved medicament dispensing device.

A further object of one embodiment of the present invention is to provide an automatic injection device for dispensing a medicament, said device having a needle connected to a barrel adapted to retain medicament and a plunger within said barrel. The device comprises an outside first body member and a second body member coaxially mounted for reciprocal movement within said outside first body member, said outside body member housing said needle, said barrel and said plunger; a first rack and a second rack housed within said outside first body member; a third rack connected to said plunger; and pinion means mounted within said outside first body member and between said first rack and said third rack and between said second rack and said third rack, said second body member including a needle cover biased to cover said needle and movable to expose said needle in use and to move said first rack and said second rack for urging said plunger into said barrel to dispense said medicament.

Another object of one embodiment of the present invention is to provide a method for dispensing a medicament from a device having a needle, connected to a barrel adapted to retain medicament and a plunger within the barrel, said device having a first body member and a second body member mounted thereon for reciprocal movement comprising the steps of providing a cover means for covering a tip of the needle; positioning the device in contact adjacent a user's skin; urging the device against the user to expose, in a first stage, the needle through the cover means; continuing to move the cover means to activate reciprocal movement of the plunger where the needle penetrates a user's skin and the medicament is forced by the plunger movement through said barrel and the needle to deliver the medicament to the user.

Having thus generally described the invention, reference will now be made to the accompanying drawings, illustrating preferred embodiments.

### BRIEF DESCRIPTION OF THE DRAWINGS

Figure 1 is a longitudinal cross section of one embodiment of the present invention before use;
Figure 2 is a view similar to Figure 1 illustrating the position of the components at a second stage;
Figure 3 is a view of the device in a fully retracted position in use;
Figure 4 is a view of the device subsequent to use; and
Figure 5 is a view of the device with components removed to illustrate a locking member.
Figure 6 is a longitudinal cross section of yet another embodiment of the device in which a needle sheath is part of a removable cap wherein the removable cap has been removed
Figure 7 is a longitudinal cross section of the device shown in Figure 6 wherein the cap is in place
Figure 8 illustrates three dimensional views of the embodiment of the device shown in Figures 6 and 7.

Similar numerals used in the drawings denote similar elements.

### DETAILED DESCRIPTION OF THE PREFERRED EMBODIMENTS

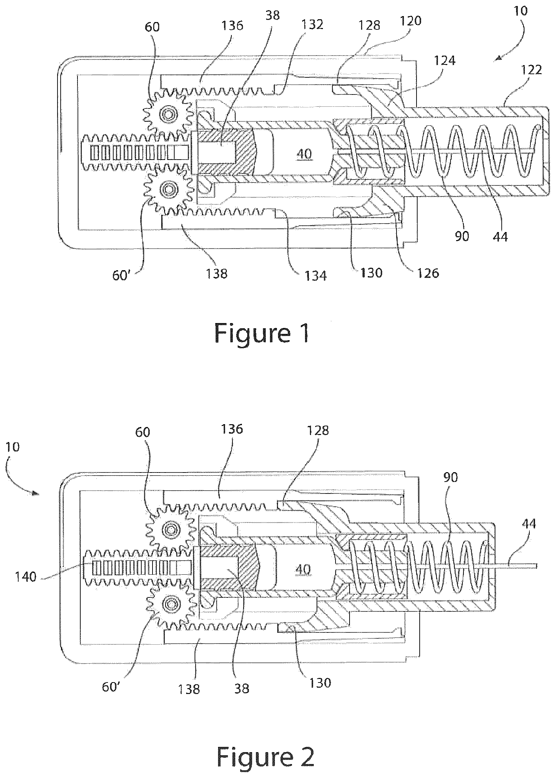

Referring now to the drawings, Figure 1 illustrates one embodiment of the present invention.

Referring now to Figure 1, shown is a longitudinal cross section of one embodiment according to the present invention. The overall arrangement is denoted by numeral 10. In this embodiment, there is an outside first body member 120, which may be formed in sections, that cooperates with a second body member 122. The outside first body member 120 may be provided with an opening to allow a user visibility of the interior to determine if the device has been discharged.

The second body member 122 is coaxially mounted within the first body member 120 for reciprocal movement. The second body member 122 houses spring 90 and needle 44 in a protective manner as is shown in the Figure, *i.e.* the needle 44 is not exposed. At a distal point from the front of second body member 122 are abutting legs 124 and 126, each of which includes a terminal end portion 128 and 130, respectively.

In use, once the second body member 122 is urged rearwardly in a direction within the first body member 120, the needle 44 is at least partially exposed and the spring 90 is at least partially compressed as is evident from Figure 2. Each of the terminal end portions 128 and 130, when in the position show in Figure 2, subsequently engages in corresponding abutments 132 and 134, respectively associated with a first rack 136 and a second rack 138 mounted within body member 120 for slidable movement therein. In this manner, corresponding abutments 132 and 134 effectively act as stops for terminal portions 128 and 130 of second body member 122.

The device 10 in this embodiment employs a rack and pinion drive mechanism. As such, rack 136 and 138 each individually engage pinion 60 and 60' as shown in the Figure.

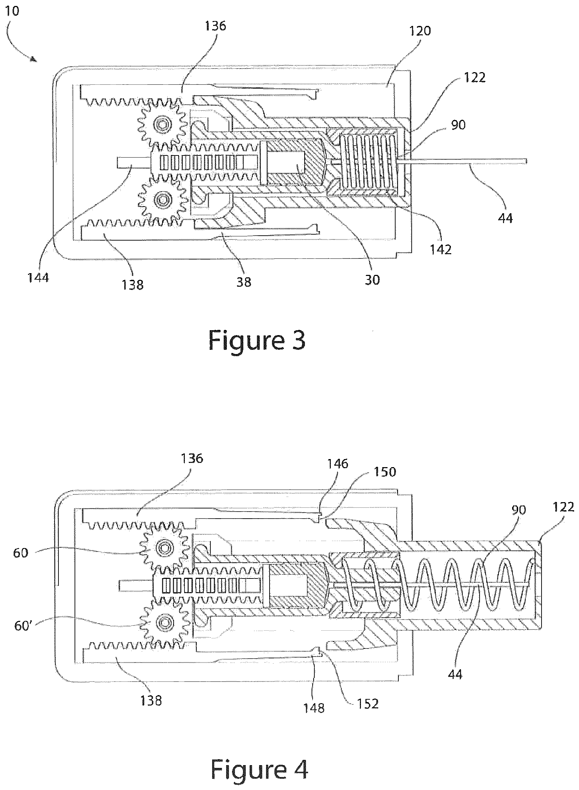

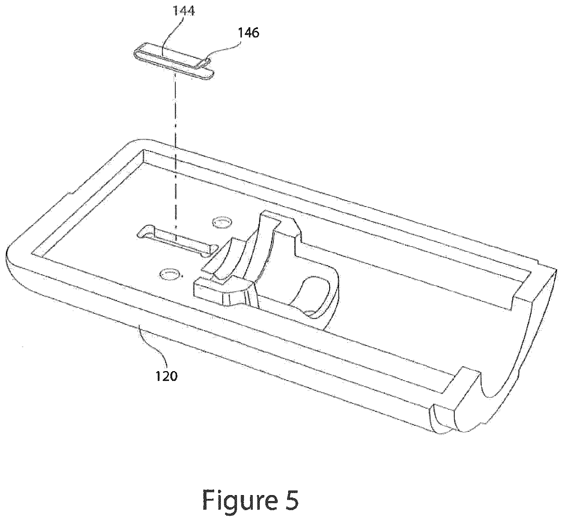

Since the abutments 132, 134 are engaged with respective terminal end portions 128 and 130 of second body member 122, the result is that further movement of second body member 122 results in the rotation of each of pinions 60 and 60' to the position shown in Figure 3. Plunger 38 is movable within barrel 40 as is typically associated with syringe arrangements. The plunger 38, in this embodiment, is operatively associated with or connect to a rack 140 which engages each of pinions 60 and 60'. The result of this mechanical union is that upon further movement of second body member 122 to the position shown in Figure 3, i.e. within body member 120, the result is that the needle 44 is fully exposed with spring 90 fully compressed and plunger 38 in the full forward position for the discharge of the medicament into a user (not shown). In this position, the spring 90 is fully compressed and is retained with a retainer 142 coaxially positioned within second body member 122. In this position, plunger 38 is locked into the position shown in Figure 3 with a lock member 144 which is housed within body member 120 rearwardly of the plunger 38. In the embodiment shown, the lock member comprises a leaf spring 144. The disposition of which is shown best in Figure 5. The lock member or leaf spring 144 includes a lip 146 which abuts the rear portion of plunger 38 when in the position of Figure 3 to prevent further use of the device 10 once the medicament has been discharged into a user (not shown). In this manner, the lock member remains in a standby position when the components within the first body member 120 are disposed in the positions shown in Figures 2 and 3.

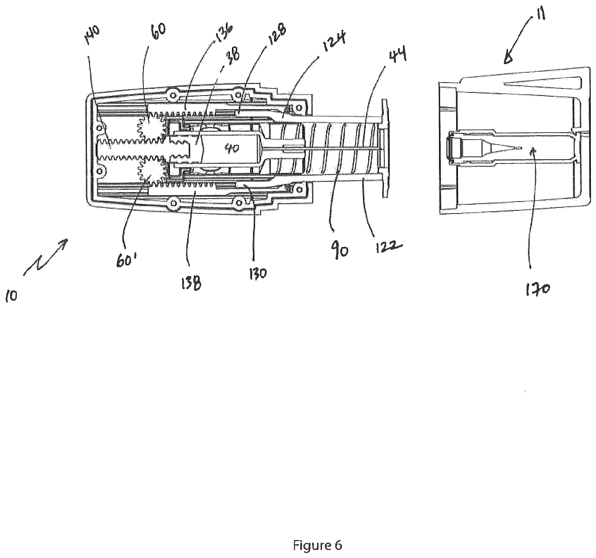

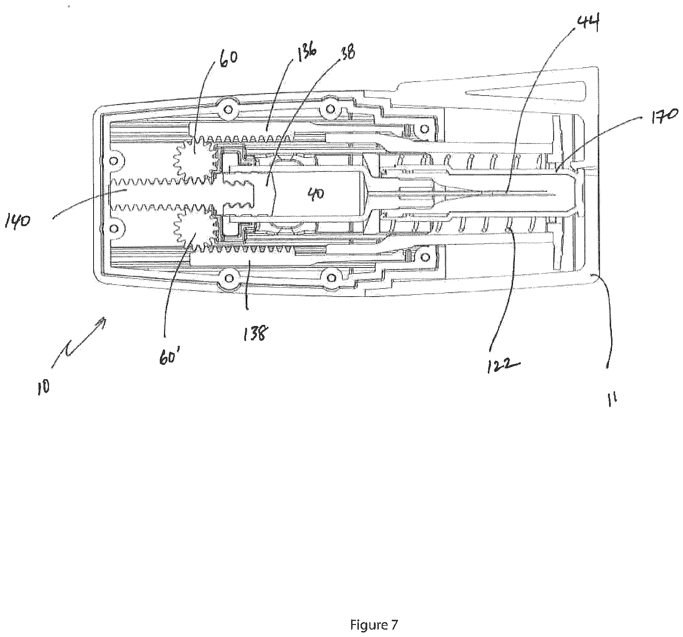

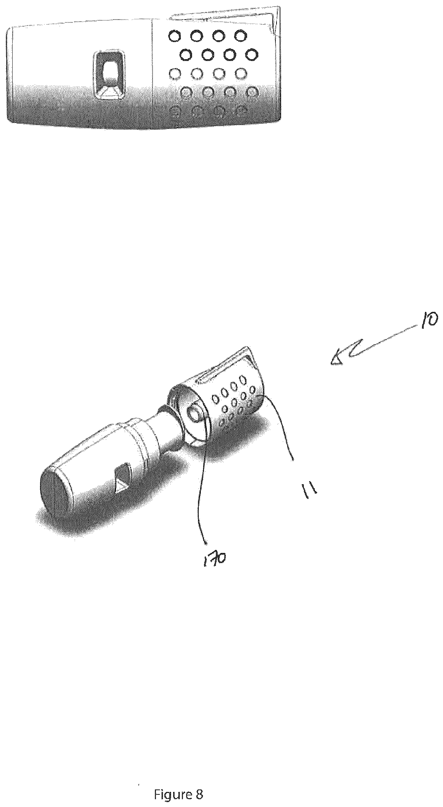

Once locked, the compression force of spring 90 is released so that the spring 90 is a relaxed state as show in Figure 4. The force that is dissipated from spring 90 then urges second body member 122 into a position where it completely surrounds and encapsulates needle 44 as shown in Figure 4. As a particular convenient feature, each rack 136 and 138 includes at respective end portion 146 and 148 lock abutments 150 and 152, respectively. The lock abutments 150, 152 are used to prevent the inadvertent movement of second body member 122 back within body member 120 in a reciprocating manner. This prevents entirely the inadvertent exposure of needle 44 so that is becomes impossible for the device 10 to be reused.
In some embodiments, the opening in the second body member 122 through which the needle 44 passes is covered with a pierceable septum which may be configured to act as a cork.
Optionally, in order to provide protection and sterility to the device 10, the same may includes a cover or cap. The cover or cap may be secured in place by mechanisms known in the art.
In some embodiments, the cover or cap includes a needle sheath such that the needle is unsheathed when the cap is removed.
Referring to Figures 6 to 8, the cap 11 includes a needle sheath 170 sized to fit within the spring 90 and protect the needle 44. In some embodiments, the sheath is further configured to act as a cork or stopper to keep the medicament inside the syringe.

## Claims

1. An automatic injection device (10) for dispensing a medicament, said device (10) having a needle (44) connected to a barrel (40) adapted to retain medicament and a plunger (38) within said barrel (40),
an outside first body member (120) and a second body member (122) coaxially mounted for reciprocal movement within said outside first body member (120) between an extended position where the needle (44) is not exposed and a compressed position where the needle (44) is exposed, said outside first body member (120) housing said needle (44), said barrel (40) and said plunger (38); **characterized in that** said device (10) comprises:
a first rack (136) and a second rack (138) housed within said outside first body member (120);
a third rack (140) connected to said plunger (38); and
pinion means (60, 60') rotatably mounted to said outside first body member (120) and between said first rack (136) and said third rack (140) and between said second rack (138) and said third rack (140), said second body member (122) including a needle cover biased with a spring (90) to cover said needle (44) in said extended position and expose said needle (44) in said compressed position; wherein in use said second body member (122) moves said first rack (136) and said second rack (138) thereby moving said plunger (38) into said barrel (40) through its connection to the rack (140) which engages each of the pinions (60, 60') to dispense said medicament, wherein said first rack (136) and said second rack (140) each include abutment means (132, 134) for abutting legs (128, 130) connected to said needle cover.

2. The automatic injection device (10) as set forth in claim 1, wherein in the fully compressed position, the spring (90) is coaxially positioned and retained with a retainer (142) within second body member (122).

3. The automatic injection device (10) as set forth in claim 1, wherein said device (10) includes lock abutments (150, 152) at respective end portion (146, 148) of each rack (136, 138) to prevent the inadvertent movement of second body member (122) back within body member (120) in a reciprocating manner.

4. The automatic injection device (10) as set forth in claim 1, wherein said device (10) includes a lock member (144) which is housed within body member (120) rearwardly of the plunger (38) to prevent further use of the device (10) once the medicament has been discharged into a user.

5. The automatic injection device (10) as set forth in claim 4, wherein said lock member (144) comprises a leaf spring.

6. The automatic injection device (10) as set forth in claim 1, wherein said first rack (136) and said second rack (138) are mounted for sliding motion within said outside first body member (120).

7. The automatic injection device (10) as set forth in claim 1, wherein said pinion means (60, 60') are mounted for rotation within said outside first body member (120).

8. The automatic injection device (10) as set forth in claim 1, wherein said device is a single use device.

9. The automatic injection device (10) as set forth in claim 1, wherein said device (10) includes a break away cap.

10. The automatic injection device (10) as set forth in claim 1, wherein said device (10) includes a removable cap (11) with needle sheath (170).

11. The automatic injection device (10) as set forth in claim 10, wherein the needle sheath (170) is further configured to act as a cork or stopper to keep the medicament inside the syringe.

## Patentansprüche

1. Automatische Injektionsvorrichtung (10) zur Abgabe eines Medikaments, wobei die Vorrichtung (10) eine Nadel (44), die mit einem Zylinder (40) verbunden ist, der zum Halten des Medikaments angepasst ist, und einen Kolben (38) in dem Zylinder (40) aufweist,
ein äußeres erstes Körperelement (120) und ein zweites Körperelement (122), das koaxial für eine Hin-und-Her-Bewegung innerhalb des äußeren ersten Körperelements (120) zwischen einer ausgefahrenen Position, in der die Nadel (44) nicht freigelegt ist, und einer komprimierten Position, in der die Nadel (44) freigelegt ist, angebracht ist, wobei das äußere erste Körperelement (120) die Nadel (44), den Zylinder (40) und den Kolben (38) aufnimmt; **dadurch gekennzeichnet, dass** die Vorrichtung (10) umfasst:
eine erste Zahnstange (136) und eine zweite Zahnstange (138), die in dem äußeren ersten Körperelement (120) untergebracht sind;
eine dritte Zahnstange (140), die mit dem Kolben (38) verbunden ist; und
Ritzelmittel (60, 60'), die drehbar an dem äußeren ersten Körperelement (120) und zwischen der ersten Zahnstange (136) und der dritten Zahnstange (140) und zwischen der zweiten Zahnstange (138) und der dritten Zahnstange (140) angebracht sind, wobei das zweite Körperelement (122) eine Nadelabdeckung aufweist, die mit einer Feder (90) vorgespannt ist, um die Nadel (44) in der ausgefahrenen Position abzudecken und die Nadel (44) in der komprimierten Position freizulegen; wobei im Gebrauch das zweite Körperelement (122) die erste Zahnstange (136) und die zweite Zahnstange (138) bewegt, wodurch der Kolben (38) in den Zylinder (40) durch seine Verbindung mit der Zahnstange (140) bewegt wird, die in jedes der Ritzel (60, 60') eingreift, um das Medikament abzugeben, wobei die erste Zahnstange (136) und die zweite Zahnstange (140) jeweils Abstützmittel (132, 134) zum Abstützen von mit der Nadelhülle verbundenen Schenkeln (128, 130) beinhalten.

2. Automatische Einspritzvorrichtung (10) nach Anspruch 1, wobei die Feder (90) in der vollständig komprimierten Position koaxial positioniert und mit einem Halter (142) innerhalb des zweiten Körperelements (122) gehalten ist.

3. Automatische Injektionsvorrichtung (10) nach Anspruch 1, wobei die Vorrichtung (10) Verriegelungsaufbauten (150, 152) am jeweiligen Endabschnitt (146, 148) jeder Zahnstange (136, 138) beinhaltet, um die unbeabsichtigte Bewegung des zweiten Körperelements (122) zurück innerhalb des Körperelements (120) hin und her zu verhindern.

4. Automatische Injektionsvorrichtung (10) nach Anspruch 1, wobei die Vorrichtung (10) ein Verriegelungselement (144) beinhaltet, das innerhalb des Körperelements (120) hinter dem Kolben (38) untergebracht ist, um eine weitere Verwendung der Vorrichtung (10) zu verhindern, sobald das Medikament in einen Benutzer abgegeben wurde.

5. Automatische Injektionsvorrichtung (10) nach Anspruch 4, wobei das Verriegelungselement (144) eine Blattfeder umfasst.

6. Automatische Injektionsvorrichtung (10) nach Anspruch 1, wobei die erste Zahnstange (136) und die zweite Zahnstange (138) zur Gleitbewegung innerhalb des äußeren ersten Körperelements (120) angebracht sind.

7. Automatische Injektionsvorrichtung (10) nach Anspruch 1, wobei die Ritzelmittel (60, 60') zur Drehung in dem äußeren ersten Körperelement (120) angebracht sind.

8. Automatische Injektionsvorrichtung (10) nach Anspruch 1, wobei die Vorrichtung eine Einwegvorrichtung ist.

9. Automatische Injektionsvorrichtung (10) nach Anspruch 1, wobei die Vorrichtung (10) eine Abreißkappe beinhaltet.

10. Automatische Injektionsvorrichtung (10) nach Anspruch 1, wobei die Vorrichtung (10) eine abnehmbare Kappe (11) mit Nadelhülle (170) beinhaltet.

11. Automatische Injektionsvorrichtung (10) nach Anspruch 10, wobei die Nadelhülle (170) weiter zum Wirken als Korken oder Stopfen konfiguriert ist, um das Medikament in der Spritze zu halten.

## Revendications

1. Dispositif d'injection automatique (10) pour distribuer un médicament, ledit dispositif (10) ayant une aiguille (44) reliée à un cylindre (40) adapté à retenir le médicament et un piston (38) à l'intérieur dudit cylindre (40),
un premier élément de corps extérieur (120) et un second élément de corps (122) montés de manière coaxiale pour un mouvement en va-et-vient dans ledit premier élément de corps extérieur (120) entre une position déployée où l'aiguille (44) n'est pas exposée et une position comprimée où l'aiguille (44) est exposée, ledit premier élément de corps extérieur (120) logeant ladite aiguille (44), ledit cylindre (40) et ledit piston (38) ; **caractérisé en ce que** ledit dispositif (10) comprend :
une première crémaillère (136) et une deuxième crémaillère (138) logées dans ledit premier élément de corps extérieur (120) ;
une troisième crémaillère (140) reliée audit piston (38) ; et
des moyens de pignon (60, 60') montés de manière rotative sur ledit premier élément de corps extérieur (120) et entre ladite première crémaillère (136) et ladite troisième crémaillère (140), et entre ladite deuxième crémaillère (138) et ladite troisième crémaillère (140), ledit second élément de corps (122) incluant un couvre-aiguille sollicité par un ressort (90) pour recouvrir ladite aiguille (44) dans ladite position déployée et exposer ladite aiguille (44) dans ladite position comprimée ; dans lequel lors de l'utilisation ledit second élément de corps (122) déplace ladite première crémaillère (136) et ladite deuxième crémaillère (138) déplaçant ainsi ledit piston (38) dans ledit cylindre (40) via sa liaison à la crémaillère (140) qui est en prise avec les pignons (60, 60') pour distribuer ledit médicament, dans lequel ladite première crémaillère (136) et ladite deuxième crémaillère (140) comportent chacune des moyens de butée (132, 134) pour des pattes de butée (128, 130) reliées audit couvre-aiguille.

2. Dispositif d'injection automatique (10) selon la revendication 1, dans lequel, dans la position complètement comprimée, le ressort (90) est positionné de manière coaxiale et retenu avec un élément de retenue (142) dans le second élément de corps (122).

3. Dispositif d'injection automatique (10) selon la revendication 1, dans lequel ledit dispositif (10) inclut des butées de blocage (150, 152) au niveau de parties d'extrémité respectives (146, 148) de chaque crémaillère (136, 138) pour empêcher le mouvement arrière involontaire du second élément de corps (122) dans l'élément de corps (120) en va-et-vient.

4. Dispositif d'injection automatique (10) selon la revendication 1, dans lequel ledit dispositif (10) inclut un élément de blocage (144) qui est logé à l'intérieur du corps (120) vers l'arrière du piston (38) pour empêcher une nouvelle utilisation du dispositif (10) une fois que le médicament a été distribué dans un utilisateur.

5. Dispositif d'injection automatique (10) selon la revendication 4, dans lequel ledit élément de blocage (144) comprend un ressort à lame.

6. Dispositif d'injection automatique (10) selon la revendication 1, dans lequel ladite première crémaillère (136) et ladite deuxième crémaillère (138) sont montées pour un mouvement coulissant à l'intérieur dudit premier élément de corps extérieur (120).

7. Dispositif d'injection automatique (10) selon la revendication 1, dans lequel lesdits moyens de pignon (60, 60') sont montés pour tourner à l'intérieur dudit premier élément de corps extérieur (120).

8. Dispositif d'injection automatique (10) selon la revendication 1, dans lequel ledit dispositif est un dispositif à usage unique.

9. Dispositif d'injection automatique (10) selon la revendication 1, dans lequel ledit dispositif (10) inclut un capuchon détachable.

10. Dispositif d'injection automatique (10) selon la revendication 1, dans lequel ledit dispositif (10) inclut un capuchon amovible (11) avec une gaine d'aiguille (170).

11. Dispositif d'injection automatique (10) selon la revendication 10, dans lequel la gaine d'aiguille (170) est en outre configurée pour servir de bouchon ou d'obturateur pour maintenir le médicament à l'intérieur de la seringue.
